# EUROPEAN PATENT SPECIFICATION

(11) **EP 1 876 989 B1**
(45) Date of publication and mention of the grant of the patent: **30.07.2014**
(21) Application number: 06752051.0
(22) Date of filing: 01.05.2006
(51) Int. Cl.: A61B 19/02

(54) **MEDICAL IMPLEMENT DISTRIBUTION AND COLLECTION SYSTEM**
MEDIZINISCHES INSTRUMENTVERTEILUNGS- UND SAMMELSYSTEM
DISTRIBUTION D'INSTRUMENTS MEDICAUX ET SYSTEME DE COLLECTE

(30) Priority: 02.05.2005 US 119960; 02.05.2005 US 119967; 02.05.2005 US 120119
(43) Date of publication of application: 16.01.2008
(60) Divisional application: 12182887.5
(73) Proprietor: Covidien AG, 8212 Neuhausen am Rheinfall (CH)
(72) Inventor: SMUDDE, Anton, M., Elk Grove Village, IL 60007 (US); BROWN, Robert, A., Algonquin, IL 60102 (US)
(74) Representative: Tomlinson, Edward James
(86) International application number: PCT/US2006/016736
(87) International publication number: WO 2006/119247

(56) References cited:
- EP-A- 1 449 491
- DE-C- 538 682
- DE-U1- 9 214 287
- GB-A- 2 275 673
- US-A- 4 809 850
- US-A- 5 251 783
- US-A- 5 706 942
- US-A1- 2003 132 129

## Description

### FIELD OF THE INVENTION

The present invention relates to a distribution and collection system for medical implements.

### BACKGROUND OF THE INVENTION

In a hospital, doctor's office or home environment setting, soiled syringes or other medical implements are commonly deposited in a disposal container following their use. Unused, sterile syringes or other medical implements are commonly obtained from a source separate from the disposal container. It is somewhat inconvenient at times, however, to provide a source of unused, sterile medical implements and a separate container for collecting the soiled medical implements following their use. In other words, it is sometimes disadvantageous from a convenience standpoint for medical practitioners to have one location from which implements are obtained and a separate location in which implements are disposed of. Attempts have been made to overcome this inconvenience. For example, improved syringe dispensing and collecting systems for personal use are disclosed in U.S. Patent No. 5,152,394, which illustrates a syringe dispensing and collecting system comprising a cylindrical container having a sterile hypodermic needle storage chamber and a separate soiled hypodermic needle collection chamber. The storage chamber is maintained in an outer peripheral region of the cylindrical container and the collection chamber is maintained in a central region of the cylindrical container. The storage chamber and the collection chamber are separated by an inner cylindrical wall. A telescoping cover mounted to the top of the container defines an opening configured to accept a soiled hypodermic needle. An outer wall of the container provides an outlet opening for the passage of sterile hypodermic needles from the storage chamber.

Several other references disclose medical waste disposal and/or dispensing apparatus. US 2003/0132129 discloses a container having an open top for disposing used syringes and an exit opening near the bottom sized for withdrawing unused syringes. A dividing tray provides a barrier between the used syringes and the unused syringes and exit opening the two-part form of independent claim 1 is based on this document. DE 538 682 discloses a device for storing and dispensing cotton balls. Several containers containing different sized cotton balls are arranged on a rotatable tray attached to a fixed base. A waste container is also attached to the fixed base for disposing used cotton balls. GB 2 275 673 discloses a waste disposal unit for used syringes. The waste disposal unit includes a box having two apertures and separate chambers corresponding to the apertures. One aperture is relatively small and is adapted to receive the needle portion of the syringe. The other aperture is relatively large and is adapted to receive the body portion of the syringe. DE 92 14 287U is directed to a dispenser for dispensing sterile objects on a first-in-first-out basis to ensure the oldest objects are removed and used first. EP 1 449 491 discloses a medical transport carrier including a carrier tray and a waste disposal container. The carrier tray includes a supply area for containing supplies, and the waste disposal container is removably attached to the carrier tray. US 5,706,942 discloses a surgical blade dispenser and disposal apparatus including a blade removal structure for disposing surgical blades and a blade support structure for supporting a plurality of unused surgical blades.

Nevertheless, there continues to be a need to further develop and improve disposal and collection devices for medical implements.

### SUMMARY OF THE INVENTION

According to an aspect of the invention a medical implement dispensing and disposal system is provided according to claim 1. Preferred aspects of the invention are provided according to the claims dependent from claim 1

In another aspect of the invention, there is provided a method of dispensing and disposing of medical implements according to claim 8. Further preferred aspects of the method are provided according to the dependent claims.

### BRIEF DESCRIPTION OF THE DRAWINGS

The invention is best understood from the following detailed description when read in connection with the accompanying drawings. It is emphasized that, according to common practice, the various features of the drawings are not to scale. On the contrary, the dimensions of the various features are arbitrarily expanded or reduced for clarity. Included in the drawings are the following figures:
FIG. 1 is a perspective view of an example of a medical implement dispensing and disposal system;
FIG. 2 is a perspective view of the example illustrated in FIG. 1 mounted within a closed enclosure;
FIG. 3 is a perspective view of the example illustrated in FIG. 1 mounted within an open enclosure;
FIG. 4 is a perspective view of another example of a medical implement dispensing and disposal system;
FIG. 5 is a perspective view of the example illustrated in FIG. 4 mounted within a closed enclosure;
FIG. 6 is a perspective view of the example illustrated in FIG. 4 mounted within an open enclosure;
FIG. 7 is a perspective view of an embodiment of a medical implement dispensing and disposal system according to an aspect of this invention;
FIG. 7A is a perspective view of the embodiment illustrated in FIG. 7 in conjunction with a flip lid;
FIG. 8 is a perspective view of the embodiment illustrated in FIG. 7 mounted within a closed enclosure; and
FIG. 9 is a perspective view of the embodiment illustrated in FIG. 7 mounted within an open enclosure.

### DETAILED DESCRIPTION OF THE INVENTION

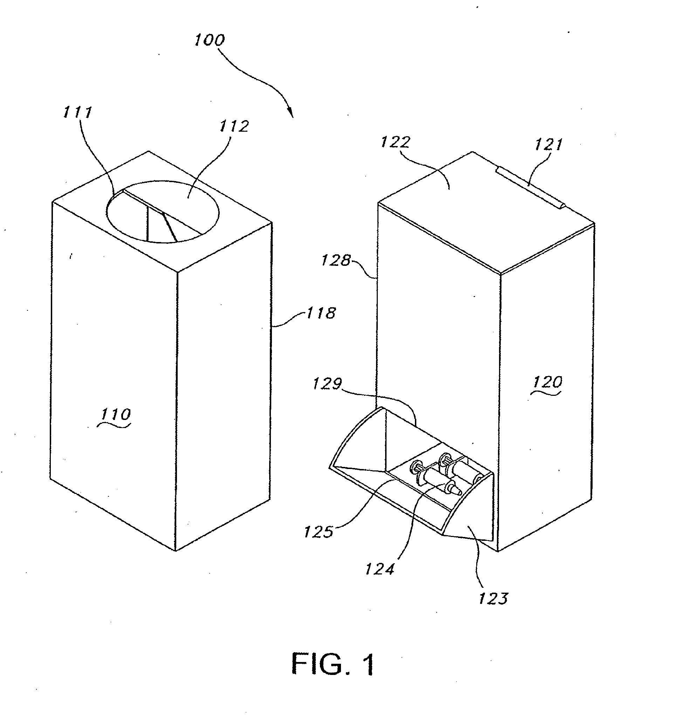

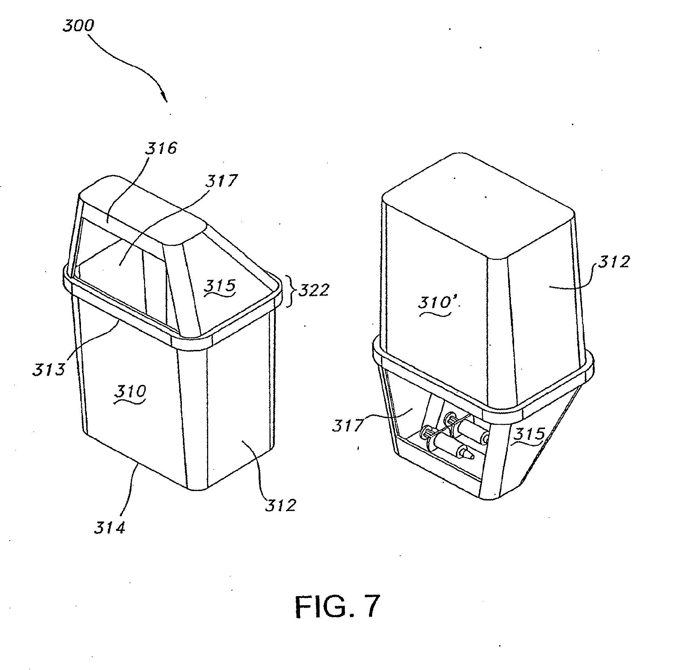

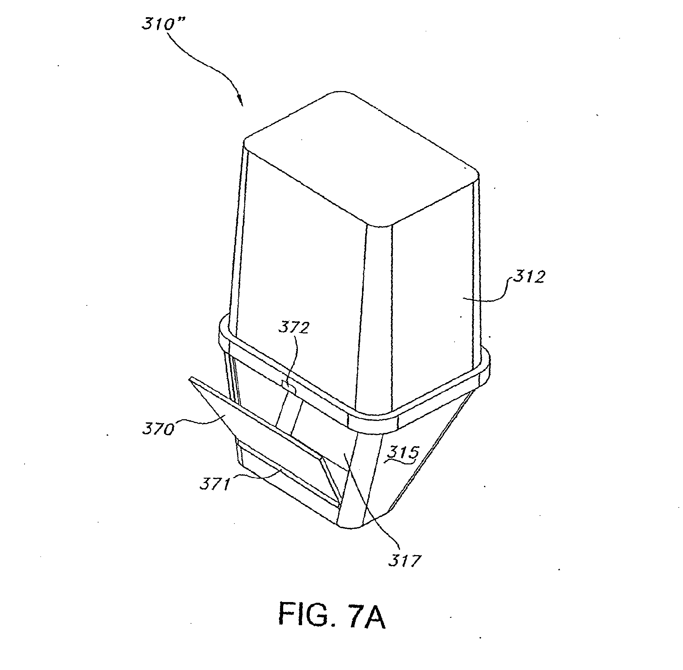

The Invention is best understood from the following detailed description when read in connection with the accompanying drawing figures, which shows in figures 1-6 examples which are not within the scope of the invention. The invention will be illustrated with reference to the figures 7-9. Such figures are intended to be illustrative rather than limiting and are included herewith to facilitate the explanation of the present invention.

As used herein, the term medical implement refers to any commonly consumed device used for medical purposes, such as but not limited to a sharp, syringe, tongue depressor, lancet, scalpel, slide, pipette and the like.

Referring generally to the figures, a medical implement dispensing and disposal system 100, 200, 300 is comprised of a medical implement dispensing container or container portion 120, 220, 310' defining a dispensing chamber; a soiled medical implement collection container or container portion 110, 210, 310 defining a collection chamber; and an optional enclosure 130, 230, 330 to house both chambers. The collection chamber defined by container 110, 210, 310 is configured to collect soiled medical implements and includes an inlet 111, 211, 317 positioned for the passage of the soiled medical implements into the collection container 110, 210, 310. The dispensing chamber defined by container 120, 220, 310' is configured to contain unused medical implements and includes an access opening 129, 223, 317 positioned for the passage of unused medical implements Into the container 120, 220, 310'.

The dispensing and collection chambers are housed within the enclosure 130, 230, 330. The enclosure 130, 230, 330 provides an opening 131, 231, 331 to access the Inlet 111, 211, 317 of the collection chamber defined by container 110, 210, 310. The enclosure 130, 230, 330 also provides an opening 132, 232, 332 to access the access opening 129, 223, 317 of the dispensing chamber defined by container 120, 220, 310'. The enclosure 130, 230, 330 includes a locking door 135, 235, 335 to inhibit unauthorized access to the dispensing container 120, 220, 310' and the collection container 110, 210, 310.

Although an enclosure 130, 230, 330 is selected for illustration, an enclosure 130, 230, 330 is an optional component of the medical implement dispensing and disposal system 100, 200, 300. A medical implement dispensing and disposal system 100, 200, 300 may include only a dispensing chamber such as the one defined by the dispensing container 120, 220, 310' and a collection chamber such as the one defined by the collection container 110, 210, 310, as illustrated in Figures 1, 4 and 7.

Referring to the example illustrated in Figures 1 through 3, more specifically Figure 1, a medical implement dispensing and disposal system is generally designated by the numeral 100. The system 100 includes a collection chamber defined by a collection container 110 and a dispensing chamber defined by a dispensing container 120. The collection container 110 includes a body portion 118, a lid 112 and an Inlet 111 formed in the lid 112. The dispensing container 120 includes a body portion 128, a lid 122, a chute 123 and an access opening 129.

The collection container 110 of the example is configured to store soiled medical implements. The walls of the collection container 110 define an interior body portion 118 and compose the structure of the collection container 110. The soiled medical implements are stored within the interior body portion 118. A lid 112 is positioned on the top wall of the collection container 110 to provide access to the body portion 118. The lid 112 defines an inlet opening 111 for passage of the soiled medical implements into the body portion 118.

The lid 112 comprises all or a portion of the top wall of the collection container 110. In this embodiment, the lid 112 includes a slideable screen that is pivotable between an open position (as illustrated in Figure 1) and a closed position. The position of the screen defines the size of the inlet opening 111 through which the soiled medical implements are passed. The user pivots the screen to dose the Inlet opening 111, for example, to restrict access to the interior body portion 118 filled with soiled medical implements. The user may also close the inlet opening 111 to prevent the leakage of soiled medical implement or contents thereof during transportation.

The lid 112 may be Integrally formed with the top wall of the collection container. Alternatively, the lid 112 may be a discrete component mounted to the top wall of the collection container as shown in Figure 1. The lid 112 can be mounted to the top wall of the collection container 110 using a hinge, adhesive, weld, clip, clamp or any other mechanical fastening method commonly known in the art. Although the lid 112 and integral inlet 111 are positioned on the top wall of the collection container 110 in this embodiment, the lid 112 and inlet 111 could be positioned on the upper portion of any side wall of the collection container 110 or at any other location, depending on other aspects of the design.

The dispensing container 120 is configured to dispense sterile, unused medical implements 124. The walls of the dispensing container 120 define the interior body portion 128 and compose the structure of the dispensing container 120. The sterile medical implements 124 are stored within the interior body portion 128. A lid 122 is hingedly connected to the top end of the dispensing container 120 by a hinge 121 to provide access to the interior body portion 128. The lid 122 pivots between an open position and a closed position (as illustrated in Figure 1). In use, the lid 122 Is pivoted to an open position to load sterile medical Implements 124 Into the Interior body portion 128 of the dispensing container 120. The lid 122 is pivoted to a closed position, as shown, to inhibit access to the sterile medical implements 124.

An access opening 129 is formed on the lower end of a side wall to provide access to the sterile medical implements 124. An extendable chute 123 is hingedly connected to the bottom end of the dispensing container 120 by a hinge 125. The chute 123 comprises two side walls and a front wall extending between the side walls. The extendable chute 123 pivots between an open position (as illustrated in Figure 1) and a closed position. In the open position, the extendable chute 123 facilitates the controlled passage of sterile medical implements. 124 from the body portion 128 via access opening 129. In the open position, the chute 123 also forms an effective barrier to prevent the medical implements 124 from uncontrollably surging out of the access opening 129. In the closed position, the chute 123 obstructs the access opening 129, thereby preventing access to the sterile medical implements 124 within the body portion 128.

For the purposes of shipping and handling, the chute 123 and lid 122 are maintained in the closed position to prevent the escapement of pre-packaged sterile medical implements 124. The chute 123 and lid 122 are also maintained in the closed position to reduce the overall shipping size of the dispensing container 120. Although not illustrated, the chute 123 and lid 122 may incorporate locking features, to further prevent unauthorized access to the sterile medical implements 124.

In use and according to the example illustrated in Figure 1, one or more of the pre-packaged sterile medical implement(s) 124 are removed from the extended chute 123 of the dispensing container 120 for use, i.e. soiling. The formerly sterile medical implement(s), now soiled, are prepared for disposal and then Inserted into the body portion 118 via Inlet opening 111. After the body portion 118 of the collection container 110 is filled to capacity, the lid 112 is closed to prevent access to the body portion 118 through the inlet opening 111.

The containers 110, 120 may be formed by an injection molding process or blow molding process or any known forming process. Alternatively, the walls of the containers may be separate and adhered, welded, snapped and/or clipped together. The containers 110, 120 are desirably composed of a substantially leak resistant material such as polypropylene or polyethylene. The containers 110, 120 may be partially or completely transparent or translucent for the purpose of monitoring the level of medical implements within the containers.

Although not illustrated, a single universal container could Incorporate the features of both containers 110 and 120. The universal container would provide both a lid 112 (with Inlet opening 111) and an extendable chute 123. When used as a dispensing container, the extendable chute 123 of the universal container would be extended to an open position and the lid 112 would be rotated to a closed position. When used as a collection container, the extendable chute 123 of the universal container would be retracted to a closed position and the lid 112 would be rotated to an open position. Manufacturing, inventory and/or tooling a single universal dispensing/collection container in lieu of two separate containers could represent a significant cost savings.

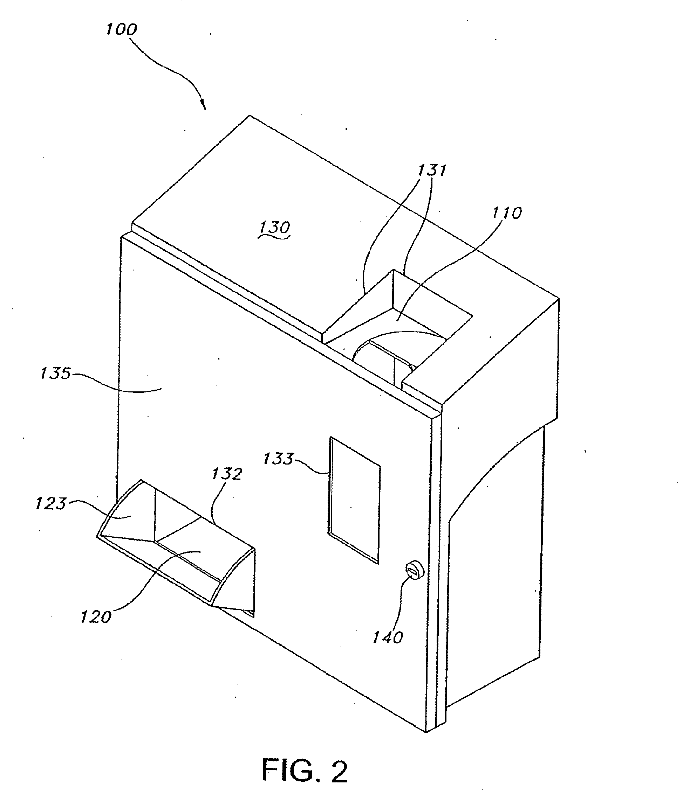

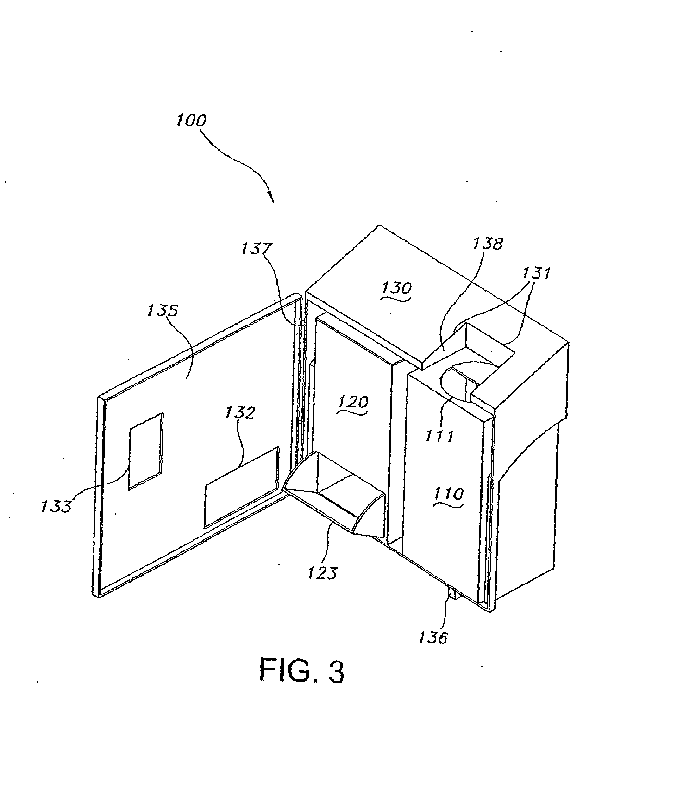

Referring specifically now to Figures 2 and 3, the medical implement dispensing and disposal system 100 illustrated in Figure 1 is mounted in an enclosure 130. As mentioned previously, although an enclosure 130 is selected for illustration and included as a component of this example , the enclosure 130 is an optional component of the dispensing and disposal system 100.

Figure 2 illustrates the medical implement dispensing and disposal system 100 maintained In an enclosure 130, wherein the door 135 of the enclosure 130 is in a closed position. Figure 3 illustrates the door 135 of the enclosure 130 In an open position. The enclosure 130 accommodates the containers 110, 120 for storage and safety purposes without inhibiting the functionality of the collection container 110 and the dispensing container 120. Accordingly, the enclosure 130 provides an opening 132 to accommodate the extendable chute 123 of the dispensing container 120. The opening 132 provides adequate clearance so that the extendable chute 123 may extend through the enclosure door 135 without obstruction (as illustrated in Figure 2).

The enclosure 130 also provides an Inlet opening 131 substantially aligned with the inlet opening 111 of the collection container 110 to permit the passage of soiled medical Implements through the inlet opening 111. The Inlet opening 131 includes a plurality of side walls 138 that extend into or toward the Interior of the enclosure 130 to contact or terminate proximal the top side of the collection container 110. The side walls 138 prohibit the soiled medical implements from unintentionally descending into the interior of the enclosure 130 or entering the chute 123.

The enclosure 130 comprises five sidewalls and a hingedly connected door 135. Although the enclosure 130 comprises five sidewalls, the enclosure may have any number of sidewalls. The door 135 is hingedly connected to a sidewall of the enclosure 130, by a hinge 137, as Illustrated in Figure 3. The door 135 may be connected to any of the sidewalls of the enclosure 130, as the orientation and position of the door 135 Is not limited to the illustration shown.

One or more supports 136 are provided on the base of the enclosure 130. The supports 136 are configured to maintain the enclosure 130 In an upright position when the enclosure 130 Is mounted on a surface such as a floor or desk. Alternatively, although not shown, the rear wall of the enclosure 130 may provide holes, slots or brackets for mounting the enclosure 130 to a wall.

A window 133 is provided on the door 135 of the enclosure 130. The window facilitates the monitoring of soiled medical implements within the collection container 110 when the door 135 is in the closed position, as illustrated in Figure 2. In use, a user monitors the level of the soiled medical implements within the collection container 110 to determine when to replace the collection container 110.

A lock 140 is provided on the door 135 of the enclosure 130 to prevent unauthorized access to the interior of the enclosure 130. The lock 140 engages with a side wall of the enclosure 130.

The enclosure 130 may be formed by an injection molding, blow molding, casting or other forming process. Alternatively, the walls of the enclosure may be separate and adhered, welded, snapped and/or clipped together. The enclosure 130 may be composed of a material such as polypropylene or polyethylene or other suitable material.

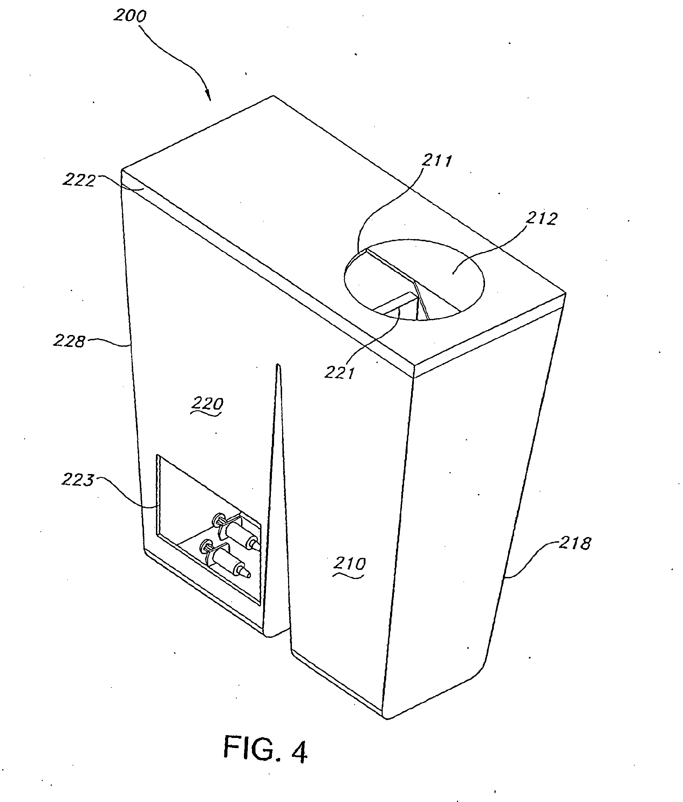

Similar to the example illustrated in Figures 1 through 3, another example of a medical implement dispensing and disposal system is illustrated in Figures 4 through 6. The medical implement dispensing and disposal system 200 includes a collection container 210 defining a collection chamber configured to collect soiled medical implements and a dispensing container 220 defining a dispensing chamber configured to dispense medical Implements. In this example the dispensing container 220 and the collection container 210 are formed from a single unitized body. Manufacturing, inventory and/or tooling of a single dispensing/collection container in lieu of two separate containers could represent a significant cost savings. The dispensing container 220 includes a body portion 228 and an access opening 223. The collection container 210 includes a body portion 218. A lid 222 is mounted to the top end of the collection container 210 and the dispensing container 220.

Similar to the example illustrated in Figures 1 through 3, the lid 222 Incorporates a slideable screen 212 that Is pivotable between an open position (as Illustrated in Figure 4) and a closed position. The position of the screen 212 defines the size of the inlet opening 211 through which the soiled medical implements are passed. The slideable screen 212 may be integral with the lid 222 or mounted (e.g. snapped or adhered) onto the lid 222 as illustrated in Figure 4.

The collection container 210 and the dispensing container 220 are formed from a single unitized body. An interior wall 221, shown as a dotted line, is positioned within the Interior of the containers and separates the collection container 210 from the dispensing container 220. The Interior wall 221 prevents the integration of the soiled and sterile medical implements. The access opening 223 accommodates the passage of sterile medical implements from the dispensing container 220. A lid 222 is provided to cover the exposed top side of the containers 210, 220. The lid 222 is mounted to the top side of the containers 210, 220 by any mechanical mounting means known in the art, e.g. tongue and groove, clips, clamps, welds, adhesive, etc.

The unitized containers 210, 220 may be formed by an injection molding process or blow molding process or other known manufacturing process. Alternatively, the walls of the containers may be separate and adhered, welded, snapped and/or clipped together. The containers 210, 220 are desirably composed of a substantially leak resistant material such as polypropylene or polyethylene. The containers 210, 220 may be partially or completely transparent or translucent for the purpose of monitoring the level of medical implements within the containers.

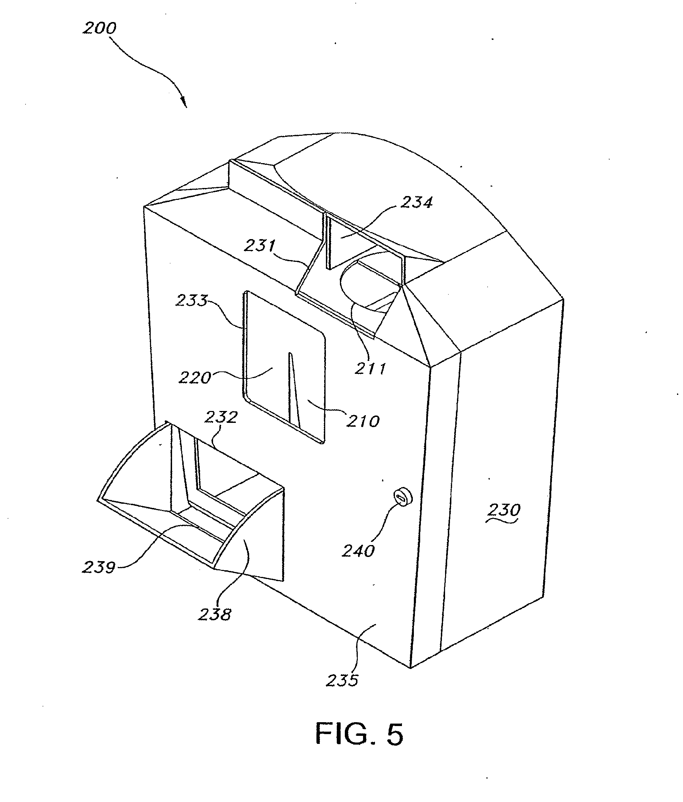

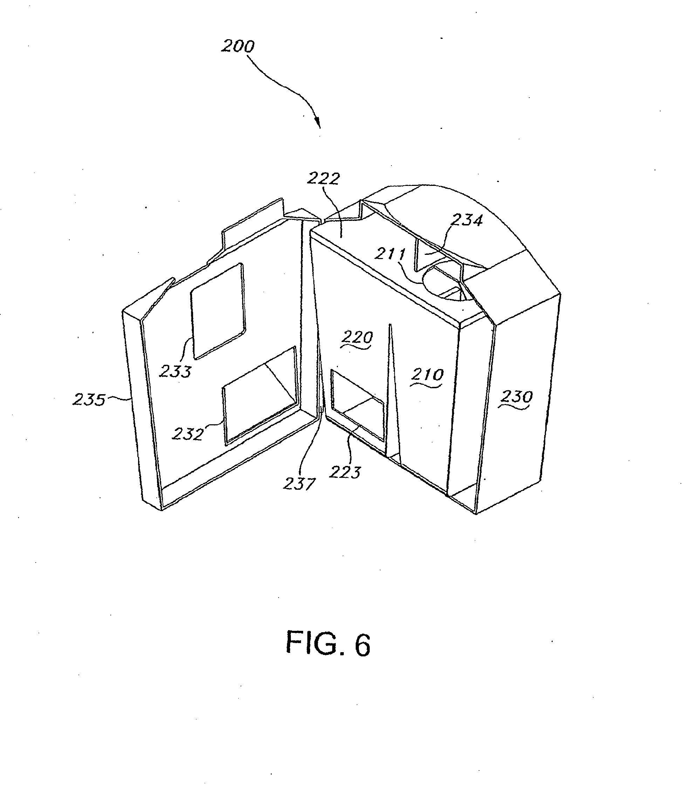

Referring specifically now to Figures 5 and 6, similar to the previous example, the medical implement dispensing and disposal system 200 illustrated in Figures 5 and 6 is mounted in an enclosure 230. Figure 5 illustrates the medical implement dispensing and disposal system 200 maintained In an enclosure 230, wherein the door 235 of the enclosure 230 is in a closed position. Figure 6 illustrates the door 235 of the enclosure 230 in an open position. The door 235 is hingedly connected to the enclosure 230 by a hinge 237. A window 233 is provided on the door 235 for monitoring the soiled medical Implements within the collection container 210. A lock 240 is also provided on the door 235 to prevent unauthorized access to the Interior portion of the enclosure 230.

Unlike the previous example however, the enclosure 230 of this example incorporates the extendable chute 238. The chute 238 is hingedly connected to the door 235 by hinge 239 and aligned with the access opening 223 of the dispensing container 220. The extendable chute 238 facilitates the controlled passage of sterile medical implements 224 through an access opening 232 of the enclosure 230 and the access opening 223 of the dispensing container 220. In the open position, the chute 238 also forms an effective barrier to prevent the medical implements 224 from uncontrollably surging out of the access opening 223 of the dispensing container 220, while concurrently providing user access to the sterile medical implements 124. In the closed position, the chute 238 obstructs the access opening 223 of the dispensing container 220, thereby prohibiting unauthorized user access to the sterile medical implements 224. In the retracted position (i.e. closed position), the side walls of the chute 238 are positioned on either side of the dispensing container 220.

The enclosure 230 provides an opening 231 substantially aligned with the inlet opening 211 of the collection container 210 to permit the passage of soiled medical implements through the inlet opening 211. The inlet 231 of the enclosure 230 includes a plurality of side walls 234 extending Into the interior of the enclosure 230. The side walls 234 are maintained in frictional contact with the lid 222 and provide a barrier to prohibit the soiled medical implements from unintentionally descending into the interior of the enclosure 230.

Similar to the examples illustrated In Figures 4 through 6, an exemplary embodiment of a medical implement dispensing and disposal system is Illustrated in Figures 7 through 9. The medical implement dispensing and disposal system 300 includes a collection container 310 defining a collection chamber configured to collect soiled medical implements and a dispensing container 310' defining a dispensing chamber configured to facilitate the distribution of medical implements. In this exemplary embodiment a single universal container is configured to be both a dispensing container 310' and a collection container 310. In one orientation the container operates as a dispensing container 310' and in another orientation the container operates as a collection container 310. Referring specifically to Figure 7, the container shown to the left Is oriented as a collection container 310 and the container shown to the right is oriented as a dispensing container 310'. The collection container 310 is oriented upright and the dispensing container 310' is oriented inverted.

The universal container 310, 310' may be advantageous from a manufacturing, inventory and/or tooling perspective. The fabrication of a single dispensing/collection container In lieu of two different containers may represent a significant cost savings.

The container 310, 310' includes a body portion 312 and a lid 315. The lid 315 is removably mounted to the top side 313 of the body portion 312. The lid 315 may be integrated with the body portion 312 or a separate component as illustrated In Figure 7. The lid 315 includes an integral flange portion 322 positioned along the periphery of the lid 315. The purposed of the flange portion 322 will be described in further detail later. The lid 315 may be composed of sheet-metal or formed by a molding process.

A barrier wall 316 formed in the lid 315 extends Into an opening 317. In the dispensing orientation (310'), the barrier wall 316 contains the sterile medical implements to facilitate the controlled passage of sterile medical implements through the opening 317 of the lid 315. The barrier wall 316 also forms an effective barrier to prevent the medical implements from uncontrollably surging out of the opening 317.

The exemplary embodiment of the dispensing container 310" Illustrated in Figure 7A includes a flip lid 370 pivotably coupled to the barrier wall 316. The flip lid 370 is configured to pivot between an open position and a closed position. The flip lid 370 illustrated in Figure 7A is shown In a partially open position. Although the flip lid 370 selected for illustration is incorporated with a dispensing container 310", the flip lid 370 may also be incorporated with a collection container 310. In the open position the flip lid 320 either provides access to the sterile medical implements within the dispensing container 310" or the soiled medical implements within the collection container 310. In the closed position the flip lid 370 obstructs the opening 317 thereby prohibiting access to the soiled medical Implements within the collection container 310 or the sterile medical implements within the dispensing container 310". A lock 372 Is coupled to the barrier wall 316 to prevent unauthorized access to the containers 310, 310". The lock is especially advantageous to safely obstruct the opening 317 of the collection container 310 upon handling and transportation of the container 310.

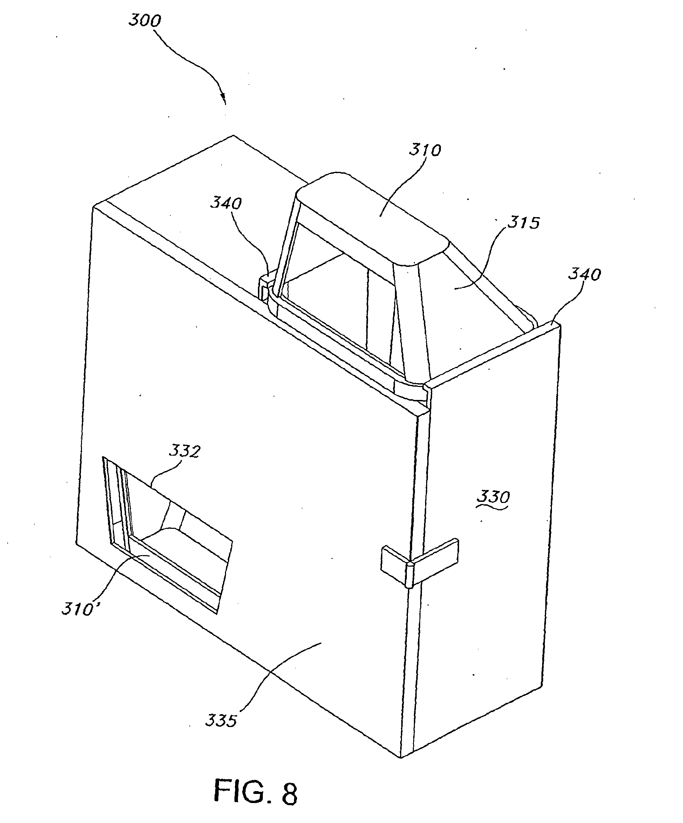

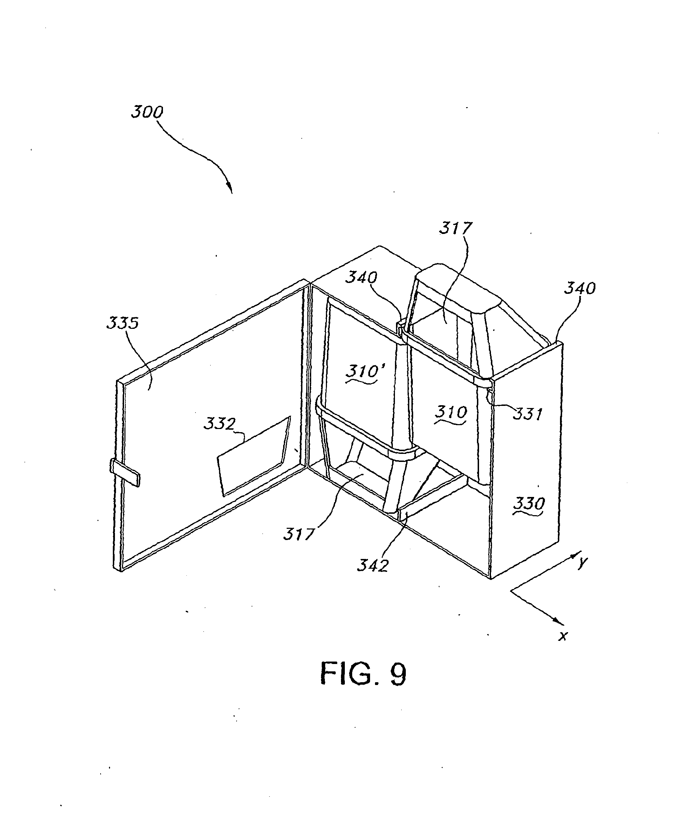

Referring specifically now to Figures 8 and 9, the medical implement dispensing and disposal system 300 illustrated in Figures 8 and 9 are accommodated In an enclosure 330. Figure 8 illustrates the medical Implement dispensing and disposal system 300 maintained in an enclosure 330, wherein the door 335 of the enclosure 330 is in a closed position. Figure 9 illustrates the door 335 of the enclosure 330 in an open position. As shown in Figure 9, the dispensing container 310' is illustrated on the left hand side of the enclosure 330 in an inverted orientation and the collection container 310 is illustrated on the right hand side of the enclosure 330 In an upright orientation.

The enclosure 330 provides an opening 331 positioned to accommodate the lid 315 of the collection container 310. The enclosure 330 also provides an access opening 332 substantially aligned with the opening 317 of the dispensing container 310' to facilitate the passage of sterile medical Implements from the dispensing container 310'. Two rail sections 340 accommodate the flange portions 322 of the collection container 310. The flange portions 322 engage with and translate along the rail sections 340. The two rail sections 340 limit the collection container 310 from shifting in the x direction. The door 335 and rear wall of the enclosure limit the collection container 310 from shifting In the y direction. A barrier 342 formed on the lower wall of the enclosure 330 limits the dispensing container 310' from shifting in the x direction. The door 335 and rear wall of the enclosure limits the dispensing container 310' from shifting in the y direction.

In use, after all of the sterile medical implements within the dispensing container 310' have been utilized and the collection container 310 Is sufficiently filled with soiled medical Implements, the filled collection container 310 is removed from the enclosure 330 and safely disposed of to provide space for an empty collection container 310. The empty dispensing container 310' is inverted to change its functionality from a dispensing container 310' to a collection container 310. The collection container 310, which was previously an empty dispensing container 310', Is mounted on the right hand side of the enclosure 330 In an upright position. A new dispensing container 310' filled with sterile medical implements is mounted on the left hand side of the enclosure 330.

Referring to the embodiment illustrated in Figures 7 through 9, the sterile medical implements may be pre-packaged within the dispensing container 310' to facilitate quick installation of the system 300. The opening 317 may be sealed with a removable barrier to prevent the escapement of the sterile medical implements from the dispensing container 310' during shipment. It is contemplated that an enclosure 330 with or without a dispensing container 310' and/or a collection container 310 could be packaged and shipped to a user. It is also contemplated that an individual dispensing container 310' and/or a collection container 310 could be packaged and shipped to a user, with or without an enclosure 330.

Although this invention has been described with reference to particular embodiments selected for illustration in the Figures 7-9, it will be appreciated that many variations and modifications can be made to the systems 100, 200, 300 and the components thereof. For example, it should be noted that it is not required that the unused medical implements are sterile, as the dispensing container is configured to hold a medical implement in any condition. Although several molding processes are mentioned, the systems and components thereof are not limited to any specific manufacturing process or material. Additionally, although the collection and dispensing containers selected for illustration are shown side by side, the collection container may be positioned above the dispensing container, or vice versa.

## Claims

1. A medical implement dispensing and disposal system (300) configured for mounting within an interior of an enclosure (330) having a first opening (331) for receiving soiled medical implements and a second opening (332) for dispensing medical implements, said medical implement dispensing and disposal system (300) comprising:
a dispensing chamber configured to be substantially enclosed within the interior of the enclosure (330) and removed from the interior of the enclosure (330), said dispensing chamber being adapted to contain medical implements and having an access opening (317) for passage of medical implements from said dispensing chamber, said access opening (317) being positioned for alignment with the second opening (332) of the enclosure (330) to facilitate passage of medical implements from the enclosure (330); and
a disposal chamber configured to be substantially enclosed within the interior of the enclosure (330) adjacent said dispensing chamber and removed from the interior of the enclosure (330), said disposal chamber being adapted to collect soiled medical implements, said disposal chamber having an inlet opening (317) for passage of soiled medical implements into said disposal chamber, said inlet opening (317) being positioned for alignment with the first opening (331) of the enclosure (330) to facilitate passage of soiled medical implements into the enclosure (330),
**characterized in that** said dispensing chamber and said disposal chamber are each defined by substantially identical containers (310, 310', 310"), said container (310, 310', 310") defining said dispensing chamber being maintained in a first orientation and said container (310, 310', 310") defining said disposal chamber being maintained in a second orientation, wherein said second orientation is inverted with respect to said first orientation.

2. The medical implement dispensing and disposal system (300) of claim 1, said containers (310, 310', 310") defining said dispensing chamber and said disposal chamber each having a lid (315).

3. The medical implement dispensing and disposal system (300) of claim 2, said lid (315) of said containers (310, 310', 310") defining said access opening (317) of said dispensing chamber in said first orientation and said lid (315) of said containers (310, 310', 310") defining said inlet opening (317) of said disposal chamber in said second orientation.

4. The medical implement dispensing and disposal system (300) of claim 1, said containers (310, 310', 310") comprising a surface (316) proximal said access opening (317) of said dispensing chamber in said first orientation, said surface (316) being configured to limit unintentional escapement of medical implements from said access opening (317) in said first orientation.

5. The medical implement dispensing and disposal system (300) of claim 4, said surface (316) of said containers (310, 310', 310") being adjacent said inlet opening (317) of said disposal chamber in said second orientation.

6. The medical implement dispensing and disposal system (300) of claim 4, said surface (316) extending upwardly when said container (310, 310', 310") is in said first orientation.

7. In combination with the medical implement dispensing and disposal system of claim 1, an enclosure accommodating said dispensing chamber and said disposal chamber.

8. A method of dispensing and disposing of medical implements comprising the steps of:
positioning a container (310, 310', 310") containing unused medical implements in an enclosure (330) in a first orientation configured to dispense the medical implements from the enclosure (330); and
reorienting the container (310, 310', 310") from the first orientation in the enclosure (330) to a second orientation in the enclosure (330) configured to receive soiled medical implements for disposal.

9. The method of claim 8, further comprising the step of obtaining unused medical implements from the container (310, 310', 310"), wherein the container (310, 310', 310") is configured to dispense unused medical implements in the first orientation and configured to collect soiled medical implements in the second orientation.

10. The method of claim 8, further comprising the step of removing unused medical implements from the container (310, 310', 310") in the first orientation for use.

11. The method of claim 8, further comprising the step of depositing used medical implements into the container (310, 310', 310") oriented in the second orientation.

12. The method of claim 8, further comprising the step of mounting a first container (310, 310', 310") in an interior of the enclosure (330), wherein the first container (310, 310', 310") is oriented in the first orientation.

13. The method of claim 12, further comprising the step of mounting a second container (310, 310', 310") in the interior of the enclosure (330) adjacent the first container (310, 310', 310"), wherein the second container (310, 310', 310") is oriented in the second orientation.

14. The method of claim 8, further comprising the step of mounting another container (310, 310', 310") containing unused medical implements in the enclosure (330)in the first orientation configured to dispense the medical implements from the enclosure (330), following the reorienting step.

15. The method of claim 8, wherein the step of reorienting the container (310, 310', 310") from the first orientation in the enclosure (330) to the second orientation in the enclosure (330) comprises inverting the container (310, 310', 310").

## Patentansprüche

1. Medizinisches Instrumentausgabe- und Entsorgungssystem (300) zur Befestigung in einem Innenraum eines Gehäuses (330) mit einer ersten Öffnung (331) zur Aufnahme verschmutzter medizinischer Instrumente und mit einer zweiten Öffnung (332) zur Ausgabe medizinischer Instrumente, wobei das medizinische Instrumentausgabe- und Entsorgungssystem (300) Folgendes umfasst:
eine Ausgabekammer, die ausgelegt ist, von dem Innenraum des Gehäuses (330) im Wesentlichen umschlossen zu werden und aus dem Innenraum des Gehäuses (330) entfernt zu werden, wobei die Ausgabekammer medizinische Instrumente enthalten kann und eine Zugangsöffnung (317) zur Durchführung von medizinischen Instrumenten aus der Ausgabekammer aufweist, wobei die Zugangsöffnung (317) zur Ausrichtung mit der zweiten Öffnung (332) des Gehäuses (330) zur Erleichterung der Durchführung von medizinischen Instrumenten aus dem Gehäuse (330) angeordnet ist; und
eine Entsorgungskammer, die ausgelegt ist, von dem Innenraum des Gehäuses (330) neben der Ausgabekammer im Wesentlichen umschlossen zu werden und aus dem Innenraum des Gehäuses (330) entfernt zu werden, wobei die Entsorgungskammer zum Sammeln von verschmutzten medizinischen Instrumenten ausgelegt ist, wobei die Entsorgungskammer eine Einlassöffnung (317) zur Durchführung von verschmutzten medizinischen Instrumenten in die Entsorgungskammer aufweist, wobei die Einlassöffnung (317) zur Ausrichtung mit der ersten Öffnung (331) des Gehäuses (330) zur Erleichterung der Durchführung von verschmutzten medizinischen Instrumenten in das Gehäuse (330) angeordnet ist,
**dadurch gekennzeichnet, dass** die Ausgabekammer und die Entsorgungskammer jeweils von im Wesentlichen
identischen Behältern (310, 310', 310") definiert werden, wobei der Behälter (310, 310', 310") die in einer ersten Orientierung gehaltene Ausgabekammer definiert und der Behälter (310, 310', 310") die in einer zweiten Orientierung gehaltene Entsorgungskammer definiert, worin die zweite Orientierung bezüglich der ersten Orientierung umgekehrt ist.

2. Medizinisches Instrumentausgabe- und Entsorgungssystem (300) nach Anspruch 1, wobei die Behälter (310, 310', 310") die Ausgabekammer und die Entsorgungskammer, die jeweils einen Deckel (315) aufweisen, definieren.

3. Medizinisches Instrumentausgabe- und Entsorgungssystem (300) nach Anspruch 2, wobei der Deckel (315) der Behälter (310, 310', 310") die Zugangsöffnung (317) der Ausgabekammer in der ersten Orientierung und der Deckel (315) der Behälter (310, 310', 310") die Einlassöffnung (317) der Entsorgungskammer in der zweiten Orientierung definiert.

4. Medizinisches Instrumentausgabe- und Entsorgungssystem (300) nach Anspruch 1, wobei die Behälter (310, 310', 310") eine Oberfläche (316) proximal zu der Zugangsöffnung (317) der Ausgabekammer in der ersten Orientierung umfassen, wobei die Oberfläche (316) zur Begrenzung eines unbeabsichtigten Entweichens von medizinischen Instrumenten aus der Zugangsöffnung (317) in der ersten Orientierung ausgelegt ist.

5. Medizinisches Instrumentausgabe- und Entsorgungssystem (300) nach Anspruch 4, wobei die Oberfläche (316) der Behälter (310, 310', 310") neben der Einlassöffnung (317) der Entsorgungskammer in der zweiten Orientierung liegt.

6. Medizinisches Instrumentausgabe- und Entsorgungssystem (300) nach Anspruch 4, wobei sich die Oberfläche (316) nach oben erstreckt, wenn sich der Behälter (310, 310', 310") in der ersten Orientierung befindet.

7. In Kombination mit dem medizinischen Instrumentausgabe- und Entsorgungssystem nach Anspruch 1, ein Gehäuse, in dem die Ausgabekammer und die Entsorgungskammer untergebracht sind.

8. Verfahren zur Ausgabe und Entsorgung von medizinischen Instrumenten, das die folgenden Schritte umfasst:
Positionierung eines Behälters (310, 310', 310"), der unbenutzte medizinischen Instrumente enthält, in einem Gehäuse (330) in einer ersten Orientierung, die zur Ausgabe der medizinischen Instrumente aus dem Gehäuse (330) ausgelegt ist; und
Umorientierung des Behälters (310, 310', 310") aus der ersten Orientierung in dem Gehäuse (330) in eine zweite Orientierung in dem Gehäuse (330), die zur Aufnahme von benutzten medizinischen Instrumenten zur Entsorgung ausgelegt ist.

9. Verfahren nach Anspruch 8, ferner umfassend den Schritt der Entnahme von unbenutzten medizinischen Instrumenten aus dem Behälter (310, 310', 310"), worin der Behälter (310, 310', 310") zur Ausgabe von unbenutzten medizinischen Instrumenten in der ersten Orientierung und zum Sammeln von verschmutzten medizinischen Instrumenten in der zweiten Orientierung ausgelegt ist.

10. Verfahren nach Anspruch 8, ferner umfassend den Schritt der Entfernung von unbenutzten medizinischen Instrumenten aus dem Behälter (310, 310', 310") in der ersten Orientierung zur Verwendung.

11. Verfahren nach Anspruch 8, ferner umfassend den Schritt der Ablage von benutzten medizinischen Instrumenten in dem Behälter (310, 310', 310"), der in der zweiten Orientierung orientiert ist.

12. Verfahren nach Anspruch 8, ferner umfassend den Schritt der Befestigung eines ersten Behälters (310, 310', 310") in einem Innenraum des Gehäuses (330), worin der erste Behälter (310, 310', 310") in der ersten Orientierung orientiert ist.

13. Verfahren nach Anspruch 12, ferner umfassend den Schritt der Befestigung eines zweiten Behälters (310, 310', 310") in dem Innenraum des Gehäuses (330) neben dem ersten Behälter (310, 310', 310"), worin der zweite Behälter (310, 310', 310") in der zweiten Orientierung orientiert ist.

14. Verfahren nach Anspruch 8, ferner umfassend den Schritt der Befestigung eines anderen Behälters (310, 310', 310"), der unbenutzte medizinische Instrumente enthält, in dem Gehäuse (330) in der erstem Orientierung, die zur Ausgabe der medizinischen Instrumente aus dem Gehäuse (330) ausgelegt ist, nach dem Umorientierungsschritt.

15. Verfahren nach Anspruch 8, worin der Schritt der Umorientierung des Behälters (310, 310', 310") aus der ersten Orientierung in dem Gehäuse (330) in die zweite Orientierung in dem Gehäuse (330) Umdrehen des Behälters (310, 310', 310") umfasst.

## Revendications

1. Système de distribution et de mise au rebut d'instruments médicaux (300), configuré pour être monté à l'intérieur d'une enceinte (330) ayant une première ouverture (331) pour recevoir des instruments médicaux souillés et une deuxième ouverture (332) pour distribuer des instruments médicaux, ledit système de distribution et de mise au rebut d'instruments médicaux (300) comprenant :
une chambre de distribution configurée de manière à être substantiellement enfermée à l'intérieur de l'enceinte (330) et à être enlevée de l'intérieur de l'enceinte (330), ladite chambre de distribution étant prévue pour contenir des instruments médicaux et ayant une ouverture d'accès (317) pour le passage d'instruments médicaux depuis ladite chambre de distribution, ladite ouverture d'accès (317) étant positionnée de manière à être alignée avec la deuxième ouverture (332) de l'enceinte (330) pour faciliter le passage d'instruments médicaux depuis l'enceinte (330) ; et
une chambre de mise au rebut configurée de manière à être substantiellement enfermée à l'intérieur de l'enceinte (330) en position adjacente à ladite chambre de distribution et à être enlevée de l'intérieur de l'enceinte (330), ladite chambre de mise au rebut étant prévue pour recueillir des instruments médicaux souillés, ladite chambre de mise au rebut ayant une ouverture d'entrée (317) pour le passage d'instruments médicaux souillés dans ladite chambre de mise au rebut, ladite ouverture d'entrée (317) étant positionnée de manière à être alignée avec la première ouverture (331) de l'enceinte (330) pour faciliter le passage d'instruments médicaux souillés dans l'enceinte (330),
**caractérisé en ce que** ladite chambre de distribution et ladite chambre de mise au rebut sont chacune définies par des récipients substantiellement identiques (310, 310', 310''), ledit récipient (310, 310', 310'') définissant ladite chambre de distribution étant maintenu dans une première orientation et ledit récipient (310, 310', 310'') définissant ladite chambre de mise au rebut étant maintenu dans une deuxième orientation, ladite deuxième orientation étant inversée par rapport à ladite première orientation.

2. Système de distribution et de mise au rebut d'instruments médicaux (300) selon la revendication 1, lesdits récipients (310, 310', 310'') définissant ladite chambre de distribution et ladite chambre de mise au rebut ayant chacun un couvercle (315).

3. Système de distribution et de mise au rebut d'instruments médicaux (300) selon la revendication 2, ledit couvercle (315) desdits récipients (310, 310', 310'') définissant ladite ouverture d'accès (317) de ladite chambre de distribution dans ladite première orientation et ledit couvercle (315) desdits récipients (310, 310', 310'') définissant ladite ouverture d'entrée (317) de ladite chambre de mise au rebut dans ladite deuxième orientation.

4. Système de distribution et de mise au rebut d'instruments médicaux (300) selon la revendication 1, lesdits récipients (310, 310', 310'') comprenant une surface (316) à proximité de ladite ouverture d'accès (317) de ladite chambre de distribution dans ladite première orientation, ladite surface (316) étant configurée de manière à limiter un échappement accidentel d'instruments médicaux hors de ladite ouverture d'accès (317) dans ladite première orientation.

5. Système de distribution et de mise au rebut d'instruments médicaux (300) selon la revendication 4, ladite surface (316) desdits récipients (310, 310', 310'') étant adjacente à ladite ouverture d'entrée (317) de ladite chambre de mise au rebut dans ladite deuxième orientation.

6. Système de distribution et de mise au rebut d'instruments médicaux (300) selon la revendication 4, ladite surface (316) s'étendant vers le haut lorsque ledit récipient (310, 310', 310'') est dans ladite première orientation.

7. Enceinte recevant ladite chambre de distribution et ladite chambre de mise au rebut, en combinaison avec le système de distribution et de mise au rebut d'instruments médicaux selon la revendication 1.

8. Procédé de distribution et de mise au rebut d'instruments médicaux, comprenant les étapes suivantes :
positionner un récipient (310, 310', 310'') contenant des instruments médicaux non utilisés dans une enceinte (330) dans une première orientation configurée de manière à distribuer des instruments médicaux depuis l'enceinte (330) ; et
réorienter le récipient (310, 310', 310'') depuis la première orientation dans l'enceinte (330) dans une deuxième orientation dans l'enceinte (330) configurée pour recevoir des instruments médicaux souillés en vue de leur mise au rebut.

9. Procédé selon la revendication 8, comprenant en outre l'étape consistant à obtenir des instruments médicaux non utilisés depuis le récipient (310, 310', 310''), le récipient (310, 310', 310'') étant configuré de manière à distribuer des instruments médicaux non utilisés dans la première orientation et étant configuré pour recueillir des instruments médicaux souillés dans la deuxième orientation.

10. Procédé selon la revendication 8, comprenant en outre l'étape consistant à enlever des instruments médicaux non utilisés hors du récipient (310, 310', 310'') dans la première orientation en vue de leur utilisation.

11. Procédé selon la revendication 8, comprenant en outre l'étape consistant à déposer des instruments médicaux utilisés dans le récipient (310, 310', 310'') orienté dans la deuxième orientation.

12. Procédé selon la revendication 8, comprenant en outre l'étape consistant à monter un premier récipient (310, 310', 310'') à l'intérieur de l'enceinte (330), le premier récipient (310, 310', 310'') étant orienté dans la première orientation.

13. Procédé selon la revendication 12, comprenant en outre l'étape consistant à monter un deuxième récipient (310, 310', 310'') à l'intérieur de l'enceinte (330) en position adjacente au premier récipient (310, 310', 310''), le deuxième récipient (310, 310', 310'') étant orienté dans la deuxième orientation.

14. Procédé selon la revendication 8, comprenant en outre l'étape consistant à monter un autre récipient (310, 310', 310'') contenant des instruments médicaux non utilisés dans l'enceinte (330) dans la première orientation, configuré pour distribuer les instruments médicaux hors de l'enceinte (330), à la suite de l'étape de réorientation.

15. Procédé selon la revendication 8, dans lequel l'étape de réorientation du récipient (310, 310', 310'') de la première orientation dans l'enceinte (330) dans la deuxième orientation dans l'enceinte (330) comprend l'inversion du récipient (310, 310', 310'').
